# EUROPÄISCHE PATENTSCHRIFT

(11) **EP 3 163 911 B1**
(45) Veröffentlichungstag und Bekanntmachung des Hinweises auf die Patenterteilung: **01.08.2018**
(21) Anmeldenummer: 16191539.2
(22) Anmeldetag: 29.09.2016
(51) Int. Cl.: H04R 25/00

(54) **HÖRGERÄTESYSTEM MIT SENSOR ZUR ERFASSUNG BIOLOGISCHER DATEN**
HEARING AID SYSTEM WITH SENSOR FOR DETECTION OF BIOLOGICAL DATA
PROTHÈSE AUDITIVE AVEC CAPTEUR POUR LA COLLECTE DE DONNÉES BIOLOGIQUES

(30) Priorität: 29.10.2015 DE 102015221187
(43) Veröffentlichungstag der Anmeldung: 03.05.2017
(73) Patentinhaber: Sivantos Pte. Ltd., Singapore 539775 (SG)
(72) Erfinder: HUSUNG, Kunibert, 91052 Erlangen (DE)
(74) Vertreter: FDST Patentanwälte

(56) Entgegenhaltungen:
- EP-A1- 2 200 342
- EP-A1- 2 736 273
- US-A1- 2013 343 585
- US-B1- 6 330 339

## Beschreibung

Ein Hörgerät dient der Versorgung einer hörgeschädigten Person mit akustischen Umgebungssignalen, die zu einer Kompensation der jeweiligen Hörschädigung entsprechend verarbeitet und insbesondere verstärkt werden. Ein Hörgerät umfasst hierzu üblicherweise einen oder mehrere akusto-elektrische Eingangswandler, beispielsweise in Form eines Mikrofons, eine Signalverarbeitungseinheit mit einem Verstärker, sowie einen elektro-akustischen Ausgangswandler. Der Ausgangswandler ist in der Regel als ein Miniaturlautsprecher realisiert und wird auch als Hörer oder Receiver bezeichnet. Er erzeugt akustische Ausgabesignale, die zum Gehör des Patienten geleitet werden und bei diesem die gewünschte Hörwahrnehmung erzeugen.

Hörgeräte gibt es in verschiedenen Ausführungen. Bei den sogenannten ITE-Hörgeräten (In-the-Ear, auch IDO bzw. In-dem-Ohr) wird ein Gehäuse, welches sämtliche funktionale Komponenten einschließlich des Mikrofons und des Receivers enthält, zumindest teilweise im Gehörgang getragen. CIC-Hörgeräte (Completely-in-Canal) sind den ITE-Hörgeräten ähnlich, werden jedoch vollständig im Gehörgang getragen. Bei BTE-Hörgeräten (Behind-the-Ear, auch Hinter-dem-Ohr bzw. HDO) wird ein Gehäuse mit Komponenten wie einer Batterie und der Signalverarbeitungseinheit hinter dem Ohr getragen. Ein flexibler, auch als Tube bezeichneter Schallschlauch leitet die akustischen Ausgabesignale des Receivers vom Gehäuse zum Gehörgang, wo üblicherweise ein entsprechendes Ohrstück am Schallschlauch zur Positionierung des Endes des Schallschlauches im Gehörgang vorgesehen ist.

Unabhängig von der Ausgestaltung des jeweiligen Hörgerätes werden mittlerweile immer häufiger Hörgeräte entwickelt, die neben ihrer klassischen Funktion als Schallverstärker für Umgebungsgeräusche auch anderweitig nutzbar sind. So können moderne Hörgeräte beispielsweise problemlos mit Telefonen, Fernsehern und/oder Musikanlagen gekoppelt werden, um deren Audiosignale direkt einzuspielen.

Ein weiteres Feld von Interesse stellt auch die Überwachung und Aufzeichnung von Vitalfunktionen des Hörgeräteträgers dar. Grundsätzlich ist eine solche Überwachung biologischer Daten, insbesondere Daten zum Stoffwechsel oder zu Vitalfunktionen, mittels sogenannter "Wearables" bekannt. Hierzu zählen beispielsweise Armbänder, Uhren, Kleidungsstücke oder Kopfhörer, die mittels Sensoren zur Messung von Blutdruck, Herzschlag oder ähnlichem genutzt werden. Die Vitalfunktionen können dann beispielsweise während einer sportlichen Aktivität direkt (bei Uhren oder Armbändern) oder (bei Kleidung, oder Kopfhörer) beispielsweise mit einem (drahtlos) gekoppelten Smartphone (bzw. der entsprechenden App) überwacht werden.

Eine Nutzung einer Hörvorrichtung gemeinsam mit einem System zur Überwachung der Aktivitäten eines Nutzers, beispielsweise während sportlicher Aktivitäten, ist aus der US 8,655,004 B2 bekannt. Das Überwachungssystem kann in der Nähe des Kopfes oder des Ohres eines Nutzers angeordnet werden und ist in der Lage, nutzerspezifische biologische Daten, wie die Körpertemperatur, den Herzschlag oder die Schweißbildung zu messen. Das Überwachungssystem umfasst hierzu einen Sensor, der entweder in die entsprechende Hörvorrichtung integriert oder an diesem befestigt werden kann.

Die US 5, 721,783 A offenbart eine Möglichkeit, mittels eines externen Gerätes, wie einer Armbanduhr oder eines Schmuckstücks, Signale aus der Umgebung oder biologische Daten des Hörgeräteträgers aufzunehmen, diese zu verstärken bzw. zu verbessern und anschließend drahtlos auf ein Hörgerät bzw. ein Ohrstück eines Hörgerätes zu übertragen.

Beiden vorbeschriebenen Systemen ist gemein, dass die Signalverarbeitung der mittels des Sensors aufgenommenen biologischen Daten über separate, eigens hierfür vorgesehene Signalverarbeitungseinheiten erfolgt, die entsprechend mit der jeweiligen Hörvorrichtung gekoppelt werden. Dies ist aufwändig und teuer.

Die US 2013/343585 A1 offenbart ein Hörhilfegerät mit einem Hörgerät sowie medizinische zur Messung von Gesundheitsparametern eines Nutzers und zur Generierung von entsprechenden Ausgabesignalen. Die Ausgabesignale der Sensoren werden gemäß der D1 über eine der Signalverarbeitungseinheit des Hörgerätes zugeführt.

Die EP 2 736 273 A1 offenbart ein Hörsystem mit einer Wahrnehmungseinheit, mittels derer mit Hilde entsprechender Sensoren beispielsweise die Körpertemperatur bestimmt und/oder EEG aufgenommen werden können. Die Sensordaten werden in der Signalverarbeitungseinheit des Hörsystems ausgewertet.

Die US 6 330 339 B1 zeigt ein Hörgerät mit einer und verschiedenen Sensoren wie Pulssensoren, Senatoren zur Messung der Gehirnströme und ähnliches. Zur Verarbeitung der Sensorsignale bzw. der entsprechenden Daten wird die Signalverarbeitungseinheit des Hörgerätes genutzt.

Aus der EP 2 200 342 A1 ist ein weiteres Hörsystem mit einer Signalverarbeitungseinheit bekannt, die zur Verarbeitung von von einem dem Hörsystem zugehörigen Gehirnstrom-Detektor übermittelten Signalen genutzt werden kann.

Der Erfindung liegt als eine erste Aufgabe zugrunde, ein Hörgerätesystem anzugeben, welches eine technisch vereinfachte Anbindung eines Sensors zur Erfassung biologischer Daten an ein Hörgerät erlaubt.

Als eine zweite Aufgabe liegt der Erfindung zugrunde, ein Verfahren anzugeben, mittels welchem eine entsprechend vereinfachte Anbindung eines Sensors zur Erfassung biologischer Daten an ein Hörgerät möglich ist.

Die erste Aufgabe der Erfindung wird erfindungsgemäß gelöst durch ein Hörgerätesystem, umfassend ein Hörgerät mit einem akusto-elektrischen Eingangswandler zur Aufnahme eines Schallsignals und zu dessen Wandlung in ein elektrisches Eingangssignal, mit einer mit dem Eingangswandler verbundenen Signalverarbeitungseinheit zur Verarbeitung des Eingangssignals und mit einem mit der Signalverarbeitungseinheit verbundenen elektro-akustischen Ausgangswandler zur Wandlung des verarbeiteten Eingangssignals in ein akustisches Ausgabesignal, sowie umfassend einen Sensor zur Erfassung eines Messwerts wenigstens einer biologischen Messgröße und zur Ausgabe eines Information über den Messwert tragenden elektrischen Informationssignals. Die zur Verarbeitung des Eingangssignals genutzte Signalverarbeitungseinheit ist hierbei signaltechnisch auch mit dem Sensor verbunden und zusätzlich zur Verarbeitung des Informationssignals eingerichtet und ausgebildet.

Die Erfindung basiert in einem ersten Schritt auf der Überlegung, dass die bislang benötigten externen Geräte zur Signalverarbeitung, Überwachung und Übertragung von Vitalfunktionen, von medizinischen Daten oder allgemein von biologischen Daten eines Hörgeräteträgers den Benutzerkomfort für den Hörgeräteträger verringern, da er zur Kopplung an das Hörgerät weitere separate Komponenten zusätzlich bei sich tragen muss. Technisch ist eine Vielzahl zusätzlicher elektronischer Bauteile notwendig, was die Ankopplung eines entsprechenden Sensors unnötig verteuert.

In einem zweiten Schritt berücksichtigt die Erfindung die Tatsache, dass in jedem Hörgerät eine Signalverarbeitungseinheit verbaut ist. Diese wird zur Verarbeitung und Verstärkung der aus den Schallsignalen generierten elektrischen Eingangssignale genutzt.

In einem dritten Schritt erkennt die Erfindung schließlich, dass die im Hörgerät eingesetzten Signalverarbeitungseinheiten grundsätzlich das Potential haben, sie zusätzlich zur Verarbeitung von weiteren und insbesondere -zur Verarbeitung von Signalen aus Sensoren zur Erfassung von biologischen Daten zu nutzen. Beispielsweise bleibt bei der Verarbeitung von Audiosignalen typischerweise ein Frequenzbereich unter- oder oberhalb des Hörvermögens außer Betracht, obwohl eine Verarbeitung derartiger Signale aufgrund der Parameter der in einer Signalverarbeitungseinheit eines Hörgeräts eingesetzten Komponenten möglich ist.

Unter Berücksichtigung dessen sieht die Erfindung vor, die Signalverarbeitungseinheit des Hörgeräts neben der Verarbeitung von Audiosignalen auch zur Verarbeitung der elektrischen Informationssignale eines Sensors zur Erfassung biologischer Daten zu nutzen. Dazu ist der entsprechende Sensor, mittels dem die jeweiligen biologischen Daten erfasst werden, signaltechnisch mit der Signalverarbeitungseinheit des Hörgeräts verbunden, so dass die Signalverarbeitungseinheit in die Lage versetzt wird, sowohl das elektrische Eingangssignals des akusto-elektrischen Eingangswandlers als auch ein elektrisches Informationssignals des Biosensors zu verarbeiten.

Mit anderen Worten erfolgt die Verarbeitung eines elektrischen Informationssignals eines Biosensors durch die Nutzung einer bereits im Hörgerät vorhandenen Signalverarbeitungseinheit. Bislang ungenutzte Kapazitäten der Signalverarbeitungseinheit können auf diese Weise effektiv genutzt werden. Auf zusätzliche elektronische Komponenten und/oder separate Signalverarbeitungseinheiten zur Anbindung eines Biosensors an ein Hörgerät kann verzichtet werden.

Der Sensor oder Biosensor erfasst einen Messwert einer biologischen Messgröße. Als Messgrößen sind beispielsweise der Puls bzw. der Herzschlag, die Körpertemperatur oder die Schweißbildung erfassbar. Auch können Sauerstoffsättigung, Blutzuckerspiegel, Blutdruck oder weitere medizinische Parameter gemessen werden. Das die Information über den Messwert tragende elektrische Informationssignal des Sensors kann eine Spannung, ein Strom, ein Widerstand, eine Kapazität, eine Induktivität oder ähnliches sein. Der Sensor gibt das die Information über den Messwert tragende elektrische Informationssignal aus, welches in der Signalverarbeitungseinheit des Hörgerätes entsprechend weiterverarbeitet wird. In einer besonders bevorzugten Ausgestaltung ist der Sensor mit einer Spannungsversorgungseinheit im Hörgerät verbunden.

Der Sensor ist in einer vorteilhaften Variante am Hörgerät angeordnet oder in das Hörgerät integriert. Insbesondere ist dabei das Hörgerät als ein ITE-Hörgerät ausgebildet. Über einen an einem Concha-Hörgerät oder an einem CIC-Hörgerät angeordneten Sensor lassen sich unmittelbar am oder im Ohr biologische Daten des Hörgeräteträgers erfassen. In einer anderen bevorzugten Ausgestaltung ist der Biosensor mit dem Hörgerät leitungsverbunden. Insbesondere ist der Sensor dabei in einem in das Ohr einsetzbaren Ohrstück des Hörgeräts montiert, das bei einem BTE-Hörgerät beispielsweise über einen Schallschlauch mit diesem verbunden ist. Bei der Variante eines BTE-Hörgeräts ist der Hörer bzw. der elektroakustische Wandler in das Ohrstück eingesetzt und ist über einen Verbindungsschlauch drahtgebunden angekoppelt. Man spricht dann auch von einem RIC-Hörgerät (Receiver-In-Canal). Diese Varianten weisen den Vorteil einer sehr einfachen Platzierung des Sensors auf. Dieser wird schlicht mit dem Hörgerät oder mit dem Ohrstück an die zum Abgriff der biologischen Daten geeignete Stelle am Körper des Hörgeräteträgers angeordnet. Eine umständliche separate Anbringung des Sensors an anderer Stelle entfällt.

In einer vorteilhaften Ausgestaltung der Erfindung umfasst die Signalverarbeitungseinheit einen Verstärker, insbesondere einen Vorverstärker, und eine dem Verstärker nachgeschaltete Prozessoreinheit, in der die eigentliche Verarbeitung des verstärkten Signals auf analoger und/oder digitaler Basis erfolgt. Verstärker und Prozessoreinheit sind übliche Bauteile einer in einem Hörgerät typischerweise verbauten Signalverarbeitungseinheit. Der Verstärker kann hierbei zur digitalen und/oder zur analogen Verstärkung des Signals ausgebildet sein. Entsprechend ist gegebenenfalls ein Analog-Digital-Wandler implementiert. Umfasst das Hörgerät eine Telespule zur induktiven Einkopplung von Audiosignalen oder zur induktiven Kopplung mit einem zweiten Hörgerät, z.B. bei einem binauralen Hörsystem, so enthält das Hörgerät in der Regel einen separaten Vorverstärker, der die eingekoppelten Signale auf das Signalniveau der über den akusto-elektrischen Eingangswandler eingekoppelten Eingangssignale hebt. In einer bevorzugten Variante ist das Informationssignal des Biosensors über den Vorverstärker der Telespule geführt, bevor es über einen Verstärker weiter verstärkt und in der Prozessoreinheit verarbeitet wird. Dies ist besonders vorteilhaft im Falle eines Biosensors, der sehr schwache Informationssignale liefert.

Grundsätzlich ist es möglich, das Informationssignal und das Eingangssignal der Signalverarbeitungseinheit, insbesondere dem Verstärker und/oder der Prozessoreinheit, getrennt voneinander zuzuführen. Von Vorteil ist es jedoch, wenn das Informationssignal aus dem Biosensor dem Eingangssignal aus dem akusto-elektrischen Eingangswandler eingangsseitig aufgeschaltet ist bzw. beide Signale gekoppelt werden, so dass die Signalverarbeitungseinheit zur gemeinsamen Verarbeitung, insbesondere zur gemeinsamen Verstärkung beider Signale genutzt ist. Bevorzugt werden zumindest beide Signale gemeinsam verstärkt. Dies ist möglich, da sich die Frequenzanteile des Audiosignals und des Informationssignals des Biosensor typischerweise unterscheiden. Gegebenenfalls ist das Informationssignal, wie erwähnt, zunächst über den Vorverstärker einer Telespule geführt, bevor es dem Eingangssignal aufgeschaltet wird.

Zweckmäßigerweise ist in der Signalverarbeitungseinheit dem Verstärker und der Prozessoreinheit eine Trenneinheit zur Trennung der gemeinsam verstärkten Signale zwischengeschaltet. Die Trenneinheit ist zu einer Trennung der mittels des Verstärkers gemeinsam verstärkten Signale eingerichtet und ausgebildet. Nach Trennung der gemeinsam verstärkten Signale werden das Audiosignal und das Informationssignal des Biosensors anschließende getrennt verarbeitet.

Bevorzugt umfasst die Prozessoreinheit zu einer getrennten Verarbeitung des Audio- bzw. Eingangssignals und des Informationssignals eine Audioprozessoreinheit zur Verarbeitung des Eingangssignals und/oder eine Bioprozessoreinheit zur Verarbeitung des Informationssignals. Die Audioprozessoreinheit übernimmt die Aufbereitung des Audiosignals für den Hörgeräteträger. Das aufbereitete Audiosignal wird entsprechend zur Ausgabe eines Schallsignals an den elektro-akustischen Ausgangswandler ausgegeben. Mittels der Bioprozessoreinheit werden die vom Biosensor ermittelten und gegebenenfalls verstärkten elektrischen Informationssignale derart verarbeitet werden, dass sie für einen Hörgeräteträger nutzbar sind. So werden die Informationssignale in einer bevorzugten Ausgestaltung beispielsweise aufbereitet, um in einer externen Ausgabeeinheit, beispielsweise einem Smartphone oder ähnlichem, angezeigt zu werden. Beispielsweise werden nach entsprechender Übermittlung der aufbereiteten Informationssignale die Vitalfunktionen des jeweiligen Nutzers graphisch (beispielsweise anhand eines Herzfrequenzsymbols auf dem Display eines Smartphones) dargestellt. Auch können Warnmeldungen aufbereitet, ausgegeben und übertragen werden. Die Verbindung der Bioprozessoreinheit mit der jeweiligen Ausgabeeinheit ist dabei vorzugsweise drahtlos, insbesondere über Bluetooth oder über WLAN, realisiert. Hierzu ist in einer vorteilhaften Variante die Bioprozessoreinheit mit einer Antenne des Hörgeräts gekoppelt, über die eine entsprechende drahtlose Verbindung mit einem externen Gerät ermöglicht ist.

Warnmeldungen, Töne oder auch Sprachansagen zu den Messwerten des Biosensors oder zu Auswertungen dazu lassen sich auch im Ausgangswandler des Hörgerätes ausgeben. Hierzu ist es in einer Ausgestaltungsvariante bevorzugt, wenn die Bioprozessoreinheit mit der Audioprozessoreinheit zu einer akustischen Signalausgabe verbunden ist. Die in der Bioprozessoreinheit verarbeiteten elektrischen Informationssignale werden für die Audioprozessoreinheit entsprechend aufbereitet, von dieser weiterverarbeitet und zur Ausgabe eines akustischen Signals an den elektro-akustischen Ausgangswandler des Hörgerätes weitergeleitet. So ist es möglich, einem Hörgeräteträger durch das Hörgerät beispielsweise dauerhaft oder bei Bedarf die Herztöne auszugeben, die Herzfrequenz oder andere biologische Parameter anzusagen oder bei körperlicher Überanstrengung bzw. bei Verlassen eines Normbereiches durch einen Signalton oder eine entsprechende Ansage direkt zu informieren bzw. zu warnen.

Die Nutzung des Verstärkers zur gemeinsamen Verstärkung des elektrischen Informationssignals des Sensors und des elektrischen Eingangssignals des akusto-elektrischen Eingangswandlers ist insbesondere dann möglich, wenn das Audiosignal und das Informationssignal des Biosensors voneinander verschiedene Frequenzbereiche aufweisen. Das Informationssignal des Sensors ist hierbei zweckmäßigerweise zeitlich variabel und umfasst im Wesentlichen Frequenzen aus einem Frequenzbereich, der unterhalb des Frequenzbereichs des Eingangssignals liegt. Bevorzugt umfasst das Informationssignal des Sensors Frequenzen in einem Frequenzbereich unterhalb von 10 Hz. Das Eingangssignal umfasst typischerweise Frequenzen in einem Frequenzbereich oberhalb von 100 Hz.

Durch die unterschiedlichen Frequenzbereiche ist zum einen ohne zusätzliche Maßnahmen eine gemeinsame Verstärkung in der Signalverarbeitungseinheit des Hörgeräts ermöglicht. Zum anderen wird eine Trennung der Signale nach erfolgter Verstärkung mittels einer Frequenzselektion möglich. Die zur Trennung eingesetzte Trenneinheit ist hierzu bevorzugt zu einer frequenzselektiven Aufspaltung bzw. Aufsplittung der gemeinsam verstärkten elektrischen Signale ausgebildet. Die Trenneinheit ist beispielsweise als eine Frequenzweiche ausgebildet und teilt das Signal in verschiedene Frequenzbereiche, also in das Informationssignal und in das Audiosignal. Die getrennten Signale werden dann getrennt in der Signalverarbeitungseinheit weiterverarbeitet, insbesondere mittels der Audioprozessoreinheit bzw. mit der Bioprozessoreinheit.

Der Sensor bzw. Biosensor ist bevorzugt als ein optischer Sensor ausgebildet und umfasst dabei zweckmäßigerweise zumindest eine Lichtquelle und eine Detektionseinheit. Als Lichtquelle ist vorzugsweise eine LED eingesetzt. Das von der LED emittierte Licht wird von der Detektionseinheit, die vorzugsweise als eine Photodiode ausgebildet ist, detektiert. Das von dem optischen Sensor detektierte Signal ist bevorzugt ein Spannungs- oder Stromsignal. Zweckmäßigerweise ist dem Sensor ein Filter nachgeschaltet, der dazu dient, die Signale zur Trennung von Stör-, Rausch- oder Untergrundsignalen vorzufiltern, bevor sie der Signalverarbeitungseinheit zugeführt werden.

Der Sensor ist besonders bevorzugt zur Erfassung des Herzschlags eines Hörgeräteträgers eingerichtet und ausgebildet. Ein zur Erfassung des Herzschlags ausgebildeter Sensor umfasst beispielsweise eine IR-LED (Infrarot-emittierende Diode), mittels derer die von einer pulsierenden Arterie reflektierten Signale gemessen werden. Mit jedem Herzschlag erweitern sich aufgrund von Veränderungen im Blutvolumen die Kapillaren und ziehen sich wieder zusammen. Das von der Haut reflektierte Licht der IR-LED erfasst diese Veränderungen. Das reflektierte Licht wird beispielsweise von einer Photodiode detektiert. Die Informationssignale werden dann wie vorbeschrieben verstärkt und weiterverarbeitet. Das Informationssignal des Sensors ist im Falle des Herzschlags ein periodisches bzw. zeitlich variables Signal.

In einer anderen Ausgestaltung wird mittels eines optischen Sensors die Sauerstoffsättigung im Blut des Hörgerätenutzers erfasst. Ein zur Erfassung der Sauerstoffsättigung im Blut ausgebildeter Sensor weist insbesondere zwei sich in ihrer Emissionswellenlänge unterscheidende Leuchtdioden (LED) auf. Als Lichtquellen für die Messung dienen insbesondere eine im sichtbaren Spektralbereich rote Leuchtdiode (R-LED) und eine infrarote Leuchtdiode (IR-LED). Auch zur Bestimmung der arteriellen Sauerstoffsättigung wird das Pulsieren des arteriellen Blutflusses genutzt, das während der Systole (Anspannungs- und dadurch Blut-Ausströmungsphase des Herzens) und der Diastole (Erschlaffungs- und somit Blut-Einströmungsphase) das Blutvolumen verändert und damit auf die Lichtabsorption einwirkt. Eine Veränderung der Lichtabsorption wird über eine Änderung im rückreflektierten Licht erfasst. Da nur die Veränderung der Lichtabsorption ausgewertet wird, haben nicht pulsierende absorbierende Stoffe wie Gewebe, Knochen und venöses Blut keine Auswirkung auf die Messung. Der Sensor misst insbesondere in der Rückreflexion das Verhältnis der roten und infraroten pulsierenden Absorption, welches in direkter Beziehung zur Sauerstoffsättigung steht. und stellt darüber die Sauerstoffsättigung dar.

In einer weiter vorteilhaften Ausgestaltung der Erfindung ist der Sensor zur Erfassung der Hautfeuchte und/oder der Körpertemperatur eines Hörgeräteträgers eingerichtet und ausgebildet. Bei einer Temperaturänderung oder erhöhter Schweißbildung ändert sich der Widerstand der Haut. Diese Widerstandsänderung wird gemessen, mittels eines entsprechenden Wandlers in ein elektrisches Signal umgewandelt und schließlich verstärkt und weiterverarbeitet. In diesem Fall ist das Informationssignal statisch. Es wird zur Weiterverarbeitung oder Verstärkung bevorzugt einem periodischen Trägersignal geeigneter Frequenz aufmoduliert.

Die zweite Aufgabe der Erfindung wird erfindungsgemäß gelöst durch ein Verfahren zur Ermittlung biologischer Daten eines Hörgeräteträgers mittels eines Hörgerätesystems, wobei mittels eines akusto-elektrischen Eingangswandlers eines Hörgeräts ein Schallsignal aufgenommen wird, wobei das Schallsignal mittels des akusto-elektrischen Eingangswandlers in ein elektrisches Eingangssignal umgewandelt wird, wobei das elektrische Eingangssignal an eine Signalverarbeitungseinheit des Hörgeräts weitergeleitet und dort verarbeitet wird, wobei das verarbeitete elektrische Eingangssignal an einen elektro-akustischen Ausgangswandler des Hörgeräts weitergeleitet und dort in ein akustisches Ausgabesignal gewandelt wird, wobei mittels eines Sensors ein Messwert wenigstens einer biologischen Messgröße erfasst wird, und wobei der Sensor ein Information über den Messwert tragendes elektrisches Informationssignal ausgibt. Hierbei wird zusätzlich auch das Informationssignal des Sensors in der zur Verarbeitung des Eingangssignals genutzten Signalverarbeitungseinheit des Hörgeräts verarbeitet.

Dabei können die für das Hörgerätesystem und dessen vorteilhaften Ausgestaltungen angegebenen Vorteile sinngemäß auf das Verfahren übertragen werden. Weitere vorteilhafte Varianten werden durch die auf das Verfahren gerichteten Unteransprüche ersichtlich.

Vorzugsweise wird das Informationssignal dem Eingangssignal eingangsseitig der Signalverarbeitungseinheit oder eines Verstärkers aufgeschaltet, und beide Signale werden in einem Verstärker der Signalverarbeitungseinheit gemeinsam verstärkt. Gegebenenfalls wird das Informationssignal zunächst in einem Vorverstärker einer Telespule vorverstärkt, ehe es dem Eingangssignal aufgeschaltet wird. Selbstverständlich ist auch eine getrennte Zufuhr der beiden Signale in den Verstärker möglich.

Das insbesondere verstärkte elektrische Eingangssignal und das insbesondere verstärkte elektrische Informationssignal werden zweckmäßigerweise in einer Prozessoreinheit verarbeitet. Die Prozessoreinheit umfasst hierbei vorzugsweise eine Audioprozessoreinheit und eine Bioprozessoreinheit, wobei die Audioprozessoreinheit das Eingangssignal und die Bioprozessoreinheit das Informationssignal verarbeitet.

In einer vorteilhaften Ausgestaltung werden die gemeinsam verstärkten Eingangs- und Informationssignals vor der Prozessoreinheit voneinander getrennt, wobei das Eingangssignal in einer Audioprozessoreinheit und das Informationssignal in einer Bioprozessoreinheit verarbeitet werden. Das in der Prozessoreinheit verarbeitete elektrische Eingangssignal wird vorzugsweise an den elektro-akustischen Ausgangswandler weitergeleitet. Das verarbeitete elektrische Informationssignal wird bevorzugt an eine insbesondere externe Ausgabeeinheit ausgegeben. Eine alternativ bevorzugte Ausgestaltung sieht vor, dass das verarbeitete elektrische Informationssignal von der Bioprozessoreinheit insbesondere über die Audioprozessoreinheit zur Ausgabe einer akustischen Information an den elektro-akustischen Ausgangswandler weitergeleitet bzw. ausgegeben wird.

Im Folgenden werden Ausführungsbeispiele der Erfindung anhand einer Zeichnung näher erläutert. Dabei zeigen:
- FIG 1: ein Hörgerätesystem mit einem Sensor zur Erfassung der Herzfrequenz eines Hörgeräteträgers, sowie
- FIG 2: ein Hörgerätesystem mit einem Sensor zur Erfassung der Sauerstoffsättigung des Blutes eines Hörgeräteträgers.

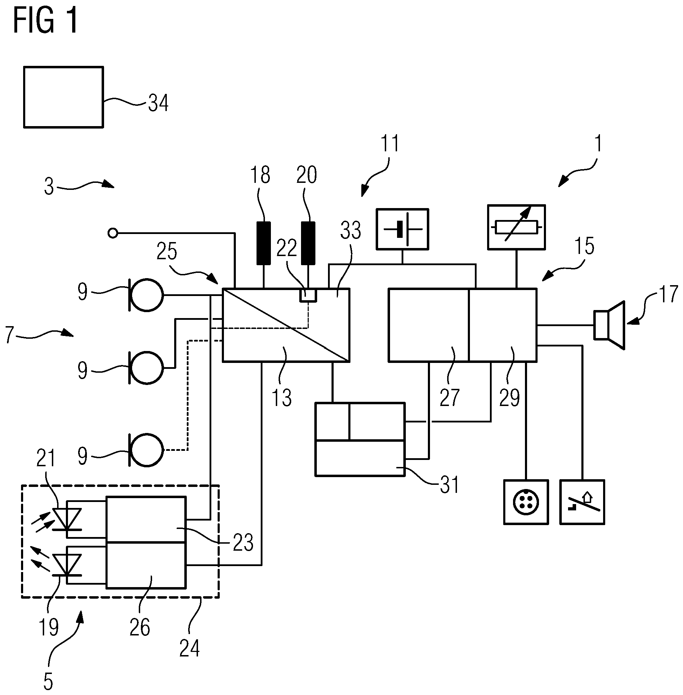

In FIG 1 ist ein Hörgerätesystem 1 mit einem Hörgerät 3 und einem optischen Sensor 5 zur Erfassung der Herzfrequenz eines Hörgeräteträgers gezeigt. Das Hörgerät 3 umfasst einen akusto-elektrischen Eingangswandler 7 mit drei Mikrophonen 9, mit denen Schallsignale insbesondere richtungsselektiv aufgenommen und in elektrische Eingangssignale gewandelt werden.

Weiter umfasst das Hörgerät 3 eine mit dem Eingangswandler 7 verbundenen Signalverarbeitungseinheit 11 mit einem vorgeschalteten Verstärker 13 und einer dem Verstärker 13 angeschlossenen Prozessoreinheit 15. Mittels des Verstärkers 13 werden die elektrischen Eingangssignale, die als Audiosignale typischerweise Frequenzen in einem Frequenzbereich oberhalb von 100 Hz aufweisen, zunächst für die weitere Verarbeitung verstärkt. In der Prozessoreinheit 15 werden die verstärkten elektrischen Eingangssignale weiterverarbeitet, insbesondere hinsichtlich des Hörvermögens des Hörgeräteträgers beispielsweise frequenzselektiv und/oder richtungsabhängig verstärkt sowie beispielsweise von Hintergrundgeräuschen und Rauschen befreit. Der Baustein 33 mit der Verstärkereinheit 13 umfasst vorliegend zugleich einen Analog-Digital-Wandler, einen Transceiver zur drahtlosen Kopplung über die Antenne 18 sowie eine Spannungsversorgungseinheit. Im Falle einer Telespule 20 ist insbesondere ein Vorverstärker 22 integriert.

Die Signalverarbeitungseinheit 11 ist ausgangsseitig mit einem elektro-akustischen Ausgangswandler 17 verbunden, der die verarbeiteten elektrischen Eingangssignale in ein akustisches Ausgabesignal wandelt, welches dann an das Gehör eines Hörgeräteträgers geleitet wird.

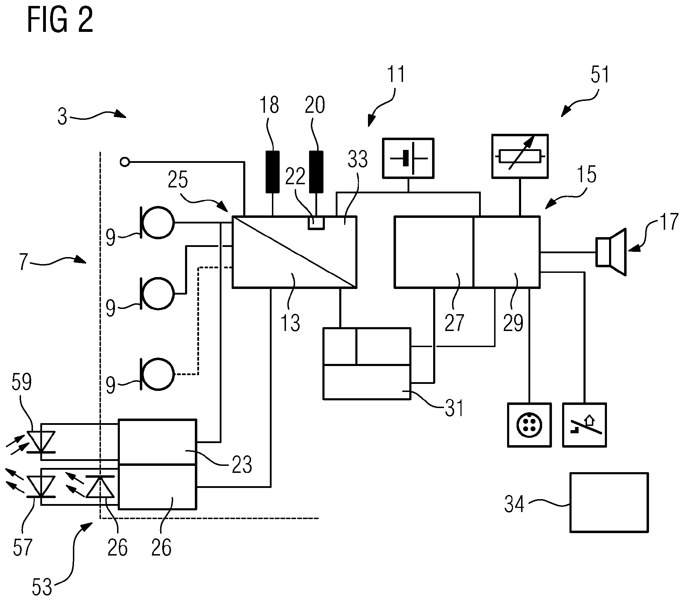

Der dem Hörgerätesystem 1 zugehörige optische Sensor 5 ist wie auch der akusto-elektrische Eingangswandler 7 des Hörgeräts mit der Signalverarbeitungseinheit 11 signaltechnisch, insbesondere drahtgebunden verbunden. Der Sensor 5 ist in diesem Fall beispielsweise unmittelbar auch am oder im Ohr des Hörgeräteträgers platziert. Insbesondere ist das Hörgerät 3 als ein ITE-Hörgerät ausgebildet, wobei der Sensor 5 unmittelbar am Hörgerät angeordnet ist. Dies ist in Fig. 2 angedeutet. In einer anderen Ausgestaltung, die in Fig. 1 angedeutet ist, ist der Sensor 5 in einem Ohrstück 24 angeordnet, wobei das Ohrstück 24 leitungsgebunden mit dem Hörgerät 3 ist und dieses als ein BTE-Hörgerät ausgebildet ist. In einer wiederum alternativen Ausgestaltung ist der Sensor 5 beispielsweise über eine Funkverbindung mit der Signalverarbeitungseinheit 11 gekoppelt. Dies ist beispielsweise über eine entsprechende Antenne 18 möglich, die mit dem Verstärker 13 verbunden ist. Der Sensor 5 ist mit einer als IR-LED ausgebildeten Lichtquelle 19 und einer Photodiode als Detektionseinheit 21 ausgebildet. Mittels des Sensors 5 wird vorliegend der Herzschlag des Hörgeräteträgers erfasst. Zur Ansteuerung der LED umfasst der Sensor 5 ein Powermanagement 26, welches über die Spannungsversorgungseinheit im Baustein 33 des Hörgeräts 3 versorgt wird.

Zur Erfassung des Herzschlages emittiert die LED als Lichtquelle19 Licht im infraroten Spektralbereich, welches bis zu einer gewissen Eindringtiefe in die Haut des Hörgeräteträgers gelangt und teilweise reflektiert wird. Die Reflexion variiert hierbei mit dem Herzschlag, da sich das Blutvolumen der Kapillaren periodisch zum Herzschlag ändert. Das reflektierte Licht wird von der Photodiode 21 detektiert. Die Photodiode 21 gibt ein sich mit dem Herzschlag periodisch veränderndes elektrisches Signal aus, das in einem Filter 23 vorgefiltert wird. Hierbei wird ein elektrisches Informationssignal mit Frequenzen unterhalb von 10 Hz, also mit der Frequenz des Herzschlags, herausgefiltert.

Das elektrische Informationssignal ist vorliegend dem elektrischen Eingangssignal an einer Eingangsseite 25 der Signalverarbeitungseinheit 11 aufgeschaltet. Beide Signale werden gemeinsam mittels des Verstärkers 13 der Signalverarbeitungseinheit 11 verstärkt. In einer anderen Variante ist das Informationssignal dem Vorverstärker 22 aufgeschaltet, bevor es gemeinsam mit dem Eingangssignal verstärkt wird. Dies ist in Fig.1 gestrichelt angedeutet. Nach der Verstärkung werden die beiden Signale in einer dem Verstärker 13 und der Prozessoreinheit 15 zwischengeschalteten Trenneinheit 27, die als eine Frequenzweiche ausgebildet ist, voneinander getrennt. Die Trenneinheit trennt hierbei das Informationssignal mit Frequenzen von weniger als 10 Hz von dem Audiosignal mit Frequenzen von mehr als 100 Hz ab.

Nach der frequenzselektiven Aufspaltung der in dem Verstärker 13 gemeinsam verstärkten elektrischen Signale, werden die verstärkten elektrischen Eingangssignale mit Frequenzen in einem Frequenzbereich oberhalb von 100 Hz einer Audioprozessoreinheit 29 der Prozessoreinheit 15 zugeführt. Hier werden die Audiosignale verarbeitet und zu einer akustischen Ausgabe an den elektro-akustischen Ausgangswandler 17 weitergeleitet.

Die verstärkten elektrischen Informationssignale des Sensors 5 mit Frequenzen in einem Frequenzbereich unterhalb von 10 Hz werden einer Bioprozessoreinheit 31 der Prozessoreinheit 15 zugeführt werden. Hier werden die Informationssignale des Sensors 5 verarbeitet und über den Transceiver im Baustein 33 mittels einer Antenne 18 drahtlos an eine externe Ausgabeeinheit 34 weitergeleitet. Die externe Ausgabeeinheit 34 ist beispielsweise ein Smartphone, auf dem die Herzfrequenz beispielsweise anhand eines Herzfrequenzsymbols mit dem gemessenen bzw. ausgewerteten Wert auf dem Display sichtbar gemacht wird.

Alternativ oder zusätzlich wird eine akustische Information über die Herzfrequenz über den elektro-akustischen Wandler 17 des Hörgerätes 3 ausgegeben. Hierzu ist die Bioprozessoreinheit 31 mit der Audioprozessoreinheit 29 verbunden. Das in der Bioprozessoreinheit 31 verarbeitete Signal wird entsprechend an die Audioprozessoreinheit 29 und von dort ebenfalls an den elektro-akustischen Ausgangswandler 17 weitergeleitet. Dieser wandelt auch das verarbeitete elektrische Informationssignal in akustische Ausgabesignale, welches dann an das Gehör eines Hörgeräteträgers geleitet wird. Das akustische Informationssignal kann die Herzfrequenz als ein periodisches Signal sein. Es kann aber auch eine Sprachausgabe über die Herzfrequenz erfolgend. Zusätzlich oder alternativ kann auch ein Warnhinweise ausgegeben werde, wenn die Herzfrequenz beispielsweise einen Norm- oder Wohlfühlbereich verlässt, den der Hörgeräteträger voreingestellt hat. Der Hörgeräteträger wird dann Warnmeldungen oder Sprachansagen bezüglich seiner Herzfrequenz direkt über das Hörgerät hören.

In FIG 2 ist ein weiteres Hörgerätesystem 51 gezeigt. Das Hörgerätesystem 51 umfasst das Hörgerät 3 gemäß FIG 1, so dass die detaillierte Beschreibung zu FIG 1 sinngemäß auf FIG 2 übertragen werden kann. Der eingesetzte Sensor 53 ist vorliegend ein optischer Sensor zur Erfassung der Sauerstoffsättigung des Blutes eines Hörgeräteträgers.

Im Unterschied zum Sensor 5 gemäß FIG 1 umfasst der vorliegende Sensor 53 zwei Lichtquellen 55, 57. Als Lichtquellen für die Messung dienen eine im sichtbaren Spektralbereich rote Leuchtdiode 55 und eine infrarote Leuchtdiode 57. Zur Bestimmung der arteriellen Sauerstoffsättigung wird das Pulsieren des arteriellen Blutflusses genutzt, das während der Systole und der Diastole das Blutvolumen verändert und damit auf die Lichtabsorption einwirkt. Da nur die Veränderung der Lichtabsorption ausgewertet wird, haben nicht pulsierende absorbierende Stoffe wie Gewebe, Knochen und venöses Blut keine Auswirkung auf die Messung.

Gemessen wird über die Reflexion die jeweilige Differenz in den Absorptionen während der Diastole und dem Spitzenwert während der Systole. Es wird hierbei postuliert, dass der Absorptionsanstieg während der Systole nur durch arterielles Blut verursacht wird. Das Messprinzip beruht darauf, dass desoxygeniertes Hämoglobin (Hb) im Infrarotbereich (= 940 nm - infrarote Leuchtdiode 57) weniger absorbiert wird, als oxygeniertes Hb bzw. oxygeniertes Hämoglobin im Rotbereich (= 660 nm - rote Leuchtdiode 55).

Die als Photodiode ausgebildete Detektionseinheit 59 des Sensors 53 misst das reflektierte Licht beider LED 55, 57. Aus dem Verhältnis der roten und infraroten pulsierenden Absorption wird die Sauerstoffsättigung ermittelt.

Die weitere Verarbeitung der Signale in der Signalverarbeitungseinheit 11 des Hörgerätes 3, sowie die Ausgabe an eine Ausgabeeinheit und/oder die die Sprachausgabe im elektro-akustischen Ausgangswandler 17 erfolgt analog zur Beschreibung gemäß FIG 1.

## Patentansprüche

1. Hörgerätesystem (1, 51), umfassend ein Hörgerät (3) mit einem akusto-elektrischen Eingangswandler (7) zur Aufnahme eines Schallsignals und zu dessen Wandlung in ein elektrisches Eingangssignal, mit einer mit dem Eingangswandler (7) verbundenen Signalverarbeitungseinheit (11) zur Verarbeitung des Eingangssignals und mit einem mit der Signalverarbeitungseinheit (11) verbundenen elektro-akustischen Ausgangswandler (17) zur Wandlung des verarbeiteten Eingangssignals in ein akustisches Ausgabesignal, sowie umfassend einen Sensor (5, 53) zur Erfassung eines Messwerts wenigstens einer biologischen Messgröße und zur Ausgabe eines Information über den Messwert tragenden elektrischen Informationssignals, wobei die zur Verarbeitung des Eingangssignals genutzte Signalverarbeitungseinheit (11) signaltechnisch auch mit dem Sensor (5, 53) verbunden ist und zusätzlich zur Verarbeitung des Informationssignals eingerichtet und ausgebildet ist, **dadurch gekennzeichnet, dass** das Informationssignal dem Eingangssignal eingangsseitig der Signalverarbeitungseinheit (11) aufgeschaltet ist.

2. Hörgerätesystem (1, 51) nach Anspruch 1, **dadurch gekennzeichnet, dass** der Sensor (5, 53) am Hörgerät (1, 51) angeordnet oder mit diesem leitungsverbunden ist, insbesondere in einem Ohrstück (24) angeordnet ist.

3. Hörgerätesystem (1, 51) nach Anspruch 1 oder 2, **dadurch gekennzeichnet, dass** die Signalverarbeitungseinheit (11) einen Verstärker (13) und eine dem Verstärker (13) nachgeschaltete Prozessoreinheit (15) umfasst.

4. Hörgerätesystem (1, 51) nach einem der vorhergehenden Ansprüche, **dadurch gekennzeichnet, dass** dem Verstärker (13) und der Prozessoreinheit (15) eine Trenneinheit (27) zwischengeschaltet ist, die zur Trennung des verstärkten Informationssignals und des verstärkten Eingangssignals eingerichtet und ausgebildet ist.

5. Hörgerätesystem (1, 51) nach Anspruch 3 oder 4, **dadurch gekennzeichnet, dass** die Prozessoreinheit (15) eine Audioprozessoreinheit (29) umfasst, die zur Verarbeitung des verstärkten Eingangssignals eingerichtet und ausgebildet ist.

6. Hörgerätesystem (1, 51) nach einem der Ansprüche 3 bis 5, **dadurch gekennzeichnet, dass** die Prozessoreinheit (15) eine Bioprozessoreinheit (31) umfasst, die zur Verarbeitung des verstärkten elektrischen Informationssignals und zu dessen Ausgabe an eine Ausgabeeinheit (34) eingerichtet und ausgebildet ist.

7. Hörgerätesystem (1, 51) nach Anspruch 6, **dadurch gekennzeichnet, dass** die Bioprozessoreinheit (31) zu einer akustischen Signalausgabe mit der Audioprozessoreinheit (29) verbunden ist.

8. Hörgerätesystem (1, 51) nach einem der vorhergehenden Ansprüche, **dadurch gekennzeichnet, dass** das Informationssignal des Sensors (5, 53) zeitlich variabel ist, wobei das Informationssignal im Wesentlichen Frequenzen aus einem Frequenzbereich unterhalb des Frequenzbereichs des Eingangssignal umfasst.

9. Hörgerätesystem (1, 51) nach Anspruch 8, **dadurch gekennzeichnet, dass** das Informationssignal des Sensors (5, 53) im Wesentlichen Frequenzen in einem Frequenzbereich unterhalb von 10 Hz umfasst.

10. Hörgerätesystem (1, 51) nach einem der vorhergehenden Ansprüche, **dadurch gekennzeichnet, dass** das Eingangssignal im Wesentlichen Frequenzen in einem Frequenzbereich oberhalb von 100 Hz umfasst.

11. Hörgerätesystem (1, 51) nach einem der Ansprüche 5 bis 10, **dadurch gekennzeichnet, dass** die Trenneinheit (27) zu einer frequenzselektiven Aufspaltung ausgebildet ist.

12. Hörgerätesystem (1, 51) nach einem der vorhergehenden Ansprüche, **dadurch gekennzeichnet, dass** der Sensor (5, 53) eine Lichtquelle (19, 55, 57) und eine Detektionseinheit (21, 59) umfasst.

13. Hörgerätesystem (1, 51) nach einem der vorhergehenden Ansprüche, **dadurch gekennzeichnet, dass** dem Sensor (5, 53) ein Filter (23) nachgeschaltet ist.

14. Hörgerätesystem (1, 51) nach einem der vorhergehenden Ansprüche, **dadurch gekennzeichnet, dass** der Sensor (5, 53) zur Erfassung eines Herzschlags und/oder einer Sauerstoffsättigung im Blut eines Hörgeräteträgers eingerichtet und ausgebildet.

15. Hörgerätesystem (1, 51) nach einem der vorhergehenden Ansprüche, **dadurch gekennzeichnet, dass** der Sensor (5, 53) zur Erfassung der Hautfeuchte und/oder der Körpertemperatur eines Hörgeräteträgers eingerichtet und ausgebildet.

16. Verfahren zur Ermittlung biologischer Daten eines Hörgeräteträgers mittels eines Hörgerätesystems (1, 51),
- wobei mittels eines akusto-elektrischen Eingangswandlers (7) eines Hörgeräts (3) ein Schallsignal aufgenommen wird,
- wobei das Schallsignal mittels des akusto-elektrischen Eingangswandlers (7) in ein elektrisches Eingangssignal umgewandelt wird,
- wobei das elektrische Eingangssignal an eine Signalverarbeitungseinheit (11) des Hörgeräts (3) weitergeleitet und dort verarbeitet wird,
- wobei das verarbeitete elektrische Eingangssignal an einen elektro-akustischen Ausgangswandler (17) des Hörgeräts (3) weitergeleitet und dort in ein akustisches Ausgabesignal gewandelt wird,
- wobei mittels eines Sensors (5, 53) ein Messwert wenigstens einer biologischen Messgröße erfasst wird, und
- wobei der Sensor (5, 53) ein Information über den Messwert tragendes elektrisches Informationssignal ausgibt,
wobei auch das Informationssignal des Sensors (5, 53) in der zur Verarbeitung des Eingangssignals genutzten Signalverarbeitungseinheit (11) verarbeitet wird, **dadurch gekennzeichnet, dass** das Informationssignal dem Eingangssignal eingangsseitig der Signalverarbeitungseinheit (11) aufgeschaltet wird.

17. Verfahren nach Anspruch 16, **dadurch gekennzeichnet, dass** das Informationssignal dem Eingangssignal eingangsseitig der Signalverarbeitungseinheit (11) aufgeschaltet wird, und beide Signale in einem Verstärker (13) der Signalverarbeitungseinheit (11) gemeinsam verstärkt werden.

18. Verfahren nach Anspruch 17, **dadurch gekennzeichnet, dass** das verstärkte elektrische Eingangssignal und das verstärkte elektrische Informationssignal in einer Prozessoreinheit (15) verarbeitet werden.

19. Verfahren nach Anspruch 18, **dadurch gekennzeichnet, dass** das verstärkte elektrische Eingangssignal und das verstärkte elektrische Informationssignal vor der Prozessoreinheit (15) voneinander getrennt werden, wobei das Eingangssignal in einer Audioprozessoreinheit (31) und das Informationssignal in einer Bioprozessoreinheit (31) verarbeitet werden..

20. Verfahren nach Anspruch 18 oder 19, **dadurch gekennzeichnet, dass** das verarbeitete Eingangssignal an den elektro-akustischen Ausgangswandler (17) weitergeleitet wird, und dass das verarbeitete Informationssignal an eine Ausgabeeinheit (34) ausgegeben wird.

21. Verfahren nach Anspruch 20, **dadurch gekennzeichnet, dass** das verarbeitete Informationssignal, insbesondere über die Audioprozessoreinheit (29), zur Ausgabe einer akustischen Information an den elektro-akustischen Ausgangswandler (17) weitergeleitet wird.

## Claims

1. Hearing device system (1, 51), comprising a hearing device (3) having an acoustoelectric input transducer (7) for picking up a sound signal and converting the latter into an electrical input signal, having a signal processing unit (11) connected to the input transducer (7) for processing the input signal and having an electroacoustic output transducer (17) connected to the signal processing unit (11) for converting the processed input signal into an audible output signal, and comprising a sensor (5, 53) for capturing a measured value of at least one biological measured variable and for outputting an electrical information signal carrying information about the measured value, wherein the signal processing unit (11) used for processing the input signal is also connected to the sensor (5, 53) for signalling purposes and is additionally set up and configured to process the information signal, **characterized in that** the information signal is connected to the input signal on the input side of the signal processing unit (11).

2. Hearing device system (1, 51) according to Claim 1, **characterized in that** the sensor (5, 53) is arranged on the hearing device (1, 51) or line-connected thereto, in particular is arranged in an earpiece (24).

3. Hearing device system (1, 51) according to Claim 1 or 2, **characterized in that** the signal processing unit (11) comprises an amplifier (13) and a processor unit (15) connected downstream of the amplifier (13).

4. Hearing device system (1, 51) according to one of the preceding claims, **characterized in that** the amplifier (13) and the processor unit (15) have a separating unit (27) connected between them that is set up and configured to separate the amplified information signal and the amplified input signal.

5. Hearing device system (1, 51) according to Claim 3 or 4, **characterized in that** the processor unit (15) comprises an audio processor unit (29) that is set up and configured to process the amplified input signal.

6. Hearing device system (1, 51) according to one of Claims 3 to 5, **characterized in that** the processor unit (15) comprises a bioprocessor unit (31) that is set up and configured to process the amplified electrical information signal and to output the latter to an output unit (34).

7. Hearing device system (1, 51) according to Claim 6, **characterized in that** the bioprocessor unit (31) is connected to the audio processor unit (29) for the purpose of audible signal output.

8. Hearing device system (1, 51) according to one of the preceding claims, **characterized in that** the information signal of the sensor (5, 53) is variable over time, the information signal substantially comprising frequencies from a frequency range below the frequency range of the input signal.

9. Hearing device system (1, 51) according to Claim 8, **characterized in that** the information signal of the sensor (5, 53) substantially comprises frequencies in a frequency range below 10 Hz.

10. Hearing device system (1, 51) according to one of the preceding claims, **characterized in that** the input signal substantially comprises frequencies in a frequency range above 100 Hz.

11. Hearing device system (1, 51) according to one of Claims 5 to 10, **characterized in that** the separating unit (27) is configured for the purpose of frequency-selective splitting.

12. Hearing device system (1, 51) according to one of the preceding claims, **characterized in that** the sensor (5, 53) comprises a light source (19, 55, 57) and a detection unit (21, 59).

13. Hearing device system (1, 51) according to one of the preceding claims, **characterized in that** the sensor (5, 53) has a filter (23) connected downstream thereof.

14. Hearing device system (1, 51) according to one of the preceding claims, **characterized in that** the sensor (5, 53) is set up and configured to capture a heartbeat and/or an oxygen saturation in the blood of a hearing device wearer.

15. Hearing device system (1, 51) according to one of the preceding claims, **characterized in that** the sensor (5, 53) is set up and configured to capture the skin moisture and/or the body temperature of a hearing device wearer.

16. Method for ascertaining biological data of a hearing device wearer by means of a hearing device system (1, 51),
- wherein an acoustoelectric input transducer (7) of a hearing device (3) is used to pick up a sound signal,
- wherein the sound signal is converted into an electrical input signal by means of the acoustoelectric input transducer (7),
- wherein the electrical input signal is forwarded to a signal processing unit (11) of the hearing device (3) and processed there,
- wherein the processed electrical input signal is forwarded to an electroacoustic output transducer (17) of the hearing device (3) and converted into an audible output signal there,
- wherein a sensor (5, 53) is used to capture a measured value of at least one biological measured variable, and
- wherein the sensor (5, 53) outputs an electrical information signal carrying information about the measured value,
wherein the information signal of the sensor (5, 53) is also processed in the signal processing unit (11) used for processing the input signal, **characterized in that** the information signal is connected to the input signal on the input side of the signal processing unit (11).

17. Method according to Claim 16, **characterized in that** the information signal is connected to the input signal on the input side of the signal processing unit (11), and the two signals are amplified together in an amplifier (13) of the signal processing unit (11).

18. Method according to Claim 17, **characterized in that** the amplified electrical input signal and the amplified electrical information signal are processed in a processor unit (15).

19. Method according to Claim 18, **characterized in that** the amplified electrical input signal and the amplified electrical information signal are separated from one another upstream of the processor unit (15), the input signal being processed in an audio processor unit (31) and the information signal being processed in a bioprocessor unit (31).

20. Method according to Claim 18 or 19, **characterized in that** the processed input signal is forwarded to the electroacoustic output transducer (17), and **in that** the processed information signal is output to an output unit (34).

21. Method according to Claim 20, **characterized in that** the processed information signal is forwarded, in particular via the audio processor unit (29), to the electroacoustic output transducer (17) for the purpose of outputting audible information.

## Revendications

1. Système d'aide auditive (1, 51), comprenant un appareil auditif (3) muni d'un convertisseur d'entrée acousto-électrique (7) destiné à acquérir un signal acoustique et à le convertir en un signal d'entrée électrique, une unité de traitement du signal (11) reliée au convertisseur d'entrée (7) et destinée à traiter le signal d'entrée et un convertisseur électro-acoustique (17) relié à l'unité de traitement du signal (11) et destiné à convertir le signal d'entrée traité en un signal de sortie acoustique, et comprenant un capteur (5, 53) destiné à détecter une valeur de mesure d'au moins une grandeur de mesure biologique et à délivrer une information concernant le signal d'information électrique transportant la valeur de mesure,
dans lequel l'unité de traitement du signal (11) utilisé pour traiter le signal d'entrée est également reliée au capteur (5, 53) et est en outre conçu et configuré pour traiter le signal d'information, **caractérisé en ce que** le signal d'information est appliqué au signal d'entrée du côté entrée de l'unité de traitement du signal (11).

2. Système d'aide auditive (1, 51) selon la revendication 1, **caractérisé en ce que** le capteur (5,53) est disposé sur l'appareil auditif (1, 51) ou est relié à celui-ci par une ligne, et est en particulier disposé dans un écouteur (24).

3. Système d'aide auditive (1, 51) selon la revendication 1, **caractérisé en ce que** l'unité de traitement du signal (11) comprend un amplificateur (13) et une unité de traitement (15) connectée en aval de l'amplificateur (13).

4. Système d'aide auditive (1, 51) selon l'une quelconque des revendications précédentes, **caractérisé en ce qu'**une unité de séparation (27) est interposée entre l'amplificateur (13) et l'unité de traitement (15) et est conçue et configurée pour séparer le signal d'information amplifié et le signal d'entrée amplifié.

5. Système d'aide auditive (1, 51) selon la revendication 3 ou 4, **caractérisé en ce que** l'unité de traitement (15) comprend une unité de traitement audio (29) conçue et configurée pour traiter le signal d'entrée amplifié.

6. Système d'aide auditive (1, 51) selon l'une des revendications 3 à 5, **caractérisé en ce que** l'unité de traitement (15) comprend une unité à bioprocesseur (31) conçue et configurée pour traiter le signal d'information électrique amplifiée et pour le délivrer à une unité de sortie (34).

7. Système d'aide auditive (1, 51) selon la revendication 6, **caractérisé en ce que** l'unité à bioprocesseur (31) est reliée à l'unité de traitement audio (29) pour une fourniture en sortie acoustique du signal.

8. Système d'aide auditive (1, 51) selon l'une des revendications précédentes, **caractérisé en ce que** le signal d'information du capteur (5, 53) est variable dans le temps, dans lequel le signal d'information comprend essentiellement des fréquences appartenant à une plage de fréquences située en dessous de la plage de fréquences du signal d'entrée.

9. Système d'aide auditive (1, 51) selon la revendication 8, **caractérisé en ce que** le signal d'information du capteur (5, 53) comprend essentiellement des fréquences se situant dans une plage de fréquences inférieure à 10 Hz.

10. Système d'aide auditive (1, 51) selon l'une des revendications précédentes, **caractérisé en ce que** le signal d'entrée comprend essentiellement des fréquences se situant dans une plage de fréquences supérieure à 100 Hz.

11. Système d'aide auditive (1, 51) selon l'une des revendications 5 à 10, **caractérisé en ce que** l'unité de séparation (27) est conçue pour effectuer un fractionnement sélectif en fréquence.

12. Système d'aide auditive (1, 51) selon l'une des revendications précédentes, **caractérisé en ce que** le capteur (5, 53) comprend une source lumineuse (19, 55, 57) est une unité de détection (21, 59).

13. Système d'aide auditive (1, 51) selon l'une des revendications précédentes, caractérisé en qu'un filtre (23) est connecté en aval du capteur (5, 53).

14. Système d'aide auditive (1, 51) selon l'une des revendications précédentes, **caractérisé en ce que** le capteur (5, 53) est conçu et réalisé pour détecter un battement cardiaque et/ou une saturation en oxygène du sang d'un porteur de l'appareil auditif.

15. Système d'aide auditive (1, 51) selon l'une des revendications précédentes, **caractérisé en ce que** le capteur (5, 53) est conçu et réalisé pour détecter l'humidité de la peau et/ou la température corporelle d'un porteur de l'appareil auditif.

16. Procédé de détermination de données biologiques d'un porteur d'appareil auditif au moyen d'un système d'aide auditive (1, 51),
- dans lequel un signal acoustique est acquis au moyen d'un convertisseur d'entrée acousto-électrique (7) d'un appareil auditif (3),
- dans lequel le signal acoustique est converti en un signal d'entrée électrique au moyen du convertisseur d'entrée acousto-électrique (7),
- dans lequel le signal d'entrée électrique est transmis à une unité de traitement du signal (11) de l'appareil auditif (3) et y est traité,
- dans lequel le signal d'entrée électrique traité est transmis à un convertisseur de sortie électro-acoustique (17) de l'appareil auditif (3) et y est converti en un signal de sortie acoustique,
- dans lequel une valeur de mesure d'au moins une grandeur de mesure biologique est détectée au moyen d'un capteur (5, 53), et
- dans lequel le capteur (5, 53) délivre une information concernant le signal d'information électrique portant la valeur de mesure, et
- dans lequel le signal d'information du capteur (5, 53) est traité dans l'unité de traitement du signal (11) utilisée pour traiter le signal d'entrée,
**caractérisé en ce que** le signal d'information est connecté au signal d'entrée du côté entrée de l'unité de traitement du signal (11).

17. Procédé selon la revendication 16, **caractérisé en ce que** le signal d'information est connecté au signal d'entrée du côté entrée de l'unité de traitement du signal (11), et **en ce que** les deux signaux sont amplifiés ensemble dans un amplificateur (13) de l'unité de traitement du signal (11).

18. Procédé selon la revendication 17, **caractérisé en ce que** le signal d'entrée électrique amplifié et le signal d'information électrique amplifié sont traités dans une unité de traitement (15).

19. Procédé selon la revendication 18, **caractérisé en ce que** le signal d'entrée électrique amplifié et le signal d'information électrique amplifié sont séparés l'un de l'autre avant l'unité de traitement (15), dans lequel le signal d'entrée est traité dans une unité de traitement audio (31) et le signal d'information est traité dans une unité à bioprocesseur (31).

20. Procédé selon la revendication 18 ou 19, **caractérisé en ce que** le signal d'entrée traité est transmis au convertisseur de sortie électro-acoustique (17) et **en ce que** le signal d'information traité est délivré à une unité de sortie (34).

21. Procédé selon la revendication 20, **caractérisé en ce que** le signal d'information traité est transmis au convertisseur de sortie électro-acoustique (17) pour la fourniture en sortie d'une information acoustique, en particulier par l'intermédiaire de l'unité de traitement audio (29).
